# EUROPÄISCHE PATENTSCHRIFT

(11) **EP 1 739 068 B1**
(45) Veröffentlichungstag und Bekanntmachung des Hinweises auf die Patenterteilung: **11.01.2012**
(21) Anmeldenummer: 06012461.7
(22) Anmeldetag: 17.06.2006
(51) Int. Cl.: C07B 41/06, C07C 45/30, C07C 205/44, C07C 269/06, C07C 47/575, C07C 47/55, C07C 47/542, C07C 47/232, C07C 49/403, C07C 47/02, B01J 23/46, B01J 35/00, B01J 23/656, B01J 23/89, B01J 21/06, B01J 37/34

(54) **Verfahren zur Ruthenium-katalysierten Oxidation von Alkoholen mit Hypochlorit**
Process for the ruthenium-catalysed oxidation of alcohols with hypochlorite
Procédé d'oxydation des alcools par l'hypochlorite en utilisant un catalyseur à base de ruthénium

(30) Priorität: 01.07.2005 DE 102005030728
(43) Veröffentlichungstag der Anmeldung: 03.01.2007
(73) Patentinhaber: Saltigo GmbH, 40764 Langenfeld (DE)
(72) Erfinder: Mägerlein, Wolfgang, Dr., 50733 Köln (DE); Köckritz, Angela, Dr., 12437 Berlin (DE); Dittmar, Andrea, Dr., 12459 Berlin (DE); Sebek, Michael, Dipl-Ing., 12359 Berlin (DE)
(74) Vertreter: Pettrich, Klaus-Günter

(56) Entgegenhaltungen:
- EP-A- 1 031 378
- JP-A- 62 265 244
- US-A- 4 647 592
- KAZUYA YAMAGUCHI: "Supported Ruthenium Catalyst for the Heterogeneous Oxidation of Alcohols with Molecular Oxygen" ANGEW. CHEM., Bd. 114, Nr. 23, 2002, Seiten 4720-4724, XP002405291
- FRIEDRICH H B ET AL: "The very efficient oxidation of alcohols by poly(4-vinylpyridine)-supported sodium ruthenate" TETRAHEDRON LETTERS, ELSEVIER, AMSTERDAM, NL, Bd. 41, Nr. 20, Mai 2000 (2000-05), Seiten 3971-3974, XP004203300 ISSN: 0040-4039
- KOCKRITZ ET AL: "Ru-catalyzed oxidation of primary alcohols" JOURNAL OF MOLECULAR CATALYSIS. A, CHEMICAL, ELSEVIER, AMSTERDAM, NL, Bd. 246, Nr. 1-2, 1. März 2006 (2006-03-01), Seiten 85-99, XP005299087 ISSN: 1381-1169
- LUCA GONSALVI ET AL.: "Selective Ruthenium-Catalyzed Oxidation of 1,2:4,5-Di-O-isopropylidene-beta-D-fructop yranose and Other Alcohols with NaOCl" ORG. LETT., Bd. 4, Nr. 10, 2002, Seiten 1659-1661, XP002405292

## Beschreibung

Die vorliegende Erfindung betrifft ein Verfahren zur Oxidation von Alkoholen zu Aldehyden oder Ketonen in Gegenwart heterogener, anorganisch geträgerter Ruthenium-Katalysatoren mit wäßriger Hypochlorit-Lösung als Oxidationsmittel.

Aldehyde und Ketone sind wichtige organische Produkte und Zwischenprodukte in der chemischen Industrie, beispielsweise für pharmazeutische und agrochemische Wirkstoffe und für Spezialchemikalien. Eine gängige Methode für die Synthese von Aldehyden bzw. Ketonen ist die Oxidation der entsprechenden primären bzw. sekundären Alkohole. Von technischer Bedeutung sind hierbei insbesondere Metall-katalysierte Oxidationsreaktionen, bei denen kostengünstige und einfach zu handhabende Oxidationsmittel verwendet werden können. Katalytische Verfahren haben gegenüber Methoden, bei denen stöchiometrische Mengen an Metallsalzen, wie z.B. Chrom(VI)-Verbindungen, Mangandioxid oder Permanganat, eingesetzt werden, erhebliche ökologische und ökonomische Vorteile. Als Aktivmetall für katalytische Alkoholoxidationen eignet sich beispielsweise Ruthenium, das gegenüber anderen, ebenfalls als Katalysator geeigneten Metallen, wie z.B. Palladium, Platin oder Osmium, wesentlich kostengünstiger bzw. weniger toxisch ist.

In Angew. Chem. 2002, 114, 4720-4724 (N. Mizuno et al.) werden Ru/Al₂O₃-Katalysatoren für die Oxidation von primären Alkoholen zu Aldehyden mit molekularem Sauerstoff beschrieben. Ferner können RuO₂-, Ru/C-, Ru/CeO₂-, Ru-Hydrotalcit-, sowie Ru-Hydroxyapatit-Katalysatoren in dieser Reaktion eingesetzt werden. Molekularer Sauerstoff weist aus sicherheitstechnischer Sicht jedoch Nachteile auf, da er in der Lage ist, entzündbare oder explosive Gemische mit anderen Stoffen zu bilden.

Von R. A. Sheldon et al, Org. Lett. 2002, 4, 1659 und Adv. Synth. Catal. 2003, 345, 1321 sowie G. Balavoine et al., J. Mol. Catal. 1985, 30, 125 sind Katalysatorsysteme bekannt, bei denen Ru-Verbindungen, wie z.B. Tetra-*n*-propylmmonium-perruthenat (*n*-Pr₄NRuO₄) oder RuCl₂(Ligand)₂ etc., in Kombination mit Natriumhypochlorit als Oxidationsmittel verwendet werden. Allerdings handelt es sich dabei um homogene Katalysatorsysteme, die gegenüber heterogenen Katalysatorsystemen dahingehend von Nachteil sind, dass die Abtrennung von den Produkten und die Recyclisierung schwieriger ist und oftmals nur geringe Selektivitäten aufweisen (vgl. Tetrahedron Letters 2000, 41, 3971-3974).

Tetrahedron Letters 2000, 41, 3971-3974 beschreibt die Oxidation von Alkoholen zu Aldehyden oder Ketonen unter Verwendung von Poly(4-vinylpyridin)-geträgerten Rutheniumkatalysatoren, wobei als mögliches Oxidationsmittel unter anderem auch NaOCl geeignet ist. Derartige polymergeträgerte Katalysatoren haben allerdings den Nachteil, dass die Trägerpolymere entweder vor Herstellung der Katalysatoren ihrerseits hergestellt werden müssen oder aber - falls kommerziell erhältlich - teurer sind als herkömmliche anorganische Trägermaterialien. Ferner können derartige organische, polymere Trägermaterialien gegen Oxidationsmittel unbeständig sein.

Es bestand somit weiterhin Bedarf an einem katalytischen Verfahren zur Oxidation von Alkoholen zu Aldehyden mit hoher Selektivität, welches die oben genannten Nachteile nicht aufweist.

JP-A-62265244 beschreibt ein Verfahren zur Oxidation von 1,4-Cyclohexandiol zu 1,4-Cyclohexandion.

Die Aufgabe der vorliegenden Erfindung bestand somit darin, ein einfaches Verfahren zur katalytischen Oxidation von Alkoholen zu Aldehyden oder Ketonen bereitzustellen, bei dem unter Einsatz von preiswerten, leicht zu handhabenden Oxidationsmitteln die gewünschten Produkte mit hoher Selektivität erhalten werden.

Es wurde nun im Rahmen der vorliegenden Erfindung gefunden, dass die Oxidation von Alkoholen auch mit heterogenen, anorganisch geträgerten Ruthenium-Katalysatoren in Gegenwart von Alkalimetall- oder Erdalkalimetall-Hypochlorit als Oxidationsmittel effizient durchgeführt werden kann. Dies ist insofern überraschend als beispielsweise aus Angew. Chem. 2002, 114, 4720-4724 bekannt ist, dass heterogene Oxidationen in der Regel aufgrund schlechter turn over numbers (TONs) und eingeschränkter Substratauswahl nachteilig sind und insbesondere nur in einer lösungsmittelfreien Variante besondere Vorteile aufweisen.

Gegenstand der vorliegenden Erfindung ist daher ein Verfahren zur Herstellung von Aldehyden der allgemeinen Formel (I), 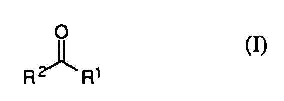 in der
R¹ ein Wasserstoffatom ist und R²
ein gegebenenfalls substituierter Kohlenwasserstoffrest, der eine Alkylgruppe, Cycloalkylgruppe, Alkenylgruppe, Arylgruppe, Arylalkylgruppe oder Arylalkenylgruppe mit jeweils 1 bis 20 Kohlenstoffatomen darstellen kann und bevorzugt gegebenenfalls mit mindestens einem Substituenten aus der Gruppe Halogenatom, Nitro, Alkoxy, Aryloxy, insbesondere Phenoxy, Aryl, Alkyl und Acyloxy substituiert sein kann,
oder eine gegebenenfalls substituierte heterocyclische Gruppe sein können, die bevorzugt mindestens ein Heteroatom aus der Gruppe Sauerstoff, Stickstoff und Schwefel besitzt,

Das Verfahren ist dadurch gekennzeichnet, dass Verbindungen der Formel (II), 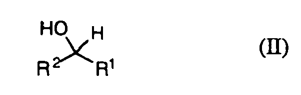 in der R¹ und R² die vorstehend genannten Bedeutungen besitzen,
mit einem Alkali- oder Erdalkalimetallhypochlorit umgesetzt werden. Der Alkohol als Substrat kann ein Monoalkohol oder ein Polyalkohol sein und es kann eine Mischung aus zwei oder mehreren dieser Alkohole eingesetzt werden. Sofern es sich bei dem oder einem der Alkohole um einen Polyalkohol handelt, können die darin enthaltenen Hydroxygruppen teilweise oder vollständig zu den entsprechenden Carbonylgruppen oxidiert werden und das resultierende Produkt eine entsprechende Polycarbonylverbindung oder eine weiterhin Hydroxygruppen enthaltende Mono- oder Polycarbonylverbindung sein. Für den Fall, dass es sich bei dem oder einem der Alkohole um einen Polyalkohol handelt, wird dieser durch die allgemeine Formel (II-a) wiedergegeben, 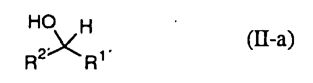 worin R¹' und R²' die für R¹ und R² genannte Bedeutung haben mit der Maßgabe, dass gegebenenfalls vorhandene Hydroxysubstituenten von R¹' und R²' in den Zielverbindungen der allgemeinen Formel (I) ganz oder teilweise zu Carbonylsubstituenten oxidiert wurden. Besonders bevorzugte Polyalkohole sind Diole, wie beispielweise gegebenenfalls substituiertes Ethylenglycol, 1,2- oder 1,3-Propandiol, 1,2-, 1,3-, 2,3- oder 1,4-Butandiol, 1,2- oder 1,5-Pentandiol, 1,2- oder 1,6-Hexandiol, 1,2- oder 1,7-Heptandiol, 1,2- oder 1,8-Octandiol, 1,2- oder 1,10-Decandiol.

Im Folgenden sind - sofern nichts anderes erwähnt - unter R¹ und R² auch die Reste R^{1'} und R^{2'} subsummiert.

Die Umsetzung erfolgt in Gegenwart eines heterogenen, anorganisch geträgerten Ruthenium-Katalysators, der gegebenenfalls zusätzlich mit einem anderen Übergangsmetall ÜM dotiert sein kann. Ein solcher heterogener, anorganisch geträgerter Ruthenium-Katalysator wird bevorzugt durch die allgemeine Formel (IIIa) oder (IIIb) repräsentiert:

Ru/Träger (IIIa)

Ru/ÜM/Träger (IIIb)

Der Rahmen der Erfindung umfasst alle oben stehenden und im Folgenden aufgeführten, allgemeinen oder in Vorzugsbereichen genannten Restedefinitionen, Parameter und Erläuterungen in beliebiger Kombination untereinander, also auch zwischen den jeweiligen Bereichen und Vorzugsbereichen.

**Aryl** steht im Rahmen der Erfindung sofern nicht gesondert vermerkt bevorzugt für carbocyclische aromatische Reste mit 6 bis 24 Gerüstkohlenstoffatomen oder heteroaromatische Reste mit 5 bis 24 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können. Weiterhin können die carbocyclischen aromatischen Reste oder heteroaromatischen Reste mit bis zu fünf gleichen oder verschiedenen Substituenten pro Cyclus substituiert sein, ausgewählt aus der Gruppe Hydroxy, Halogen, Nitro, Cyano, freies oder geschütztes Formyl, C₁-C₁₂-Alkyl, C₁-C₁₂-Halogenalkyl, C₅-C₁₄-Aryl, C₆-C₁₅-Arylalkyl, C₁-C₁₂-Alkoxy, C₁-C₁₂-Alkoxycarbonyl, gegebenenfalls substituiertes C₁-C₁₂-Alkylamino oder C₁-C₁₂-Alkoxycarbonylamino, wie z.B. tert-Butoxycarbonylamino (BOC-Amino), Carboxyl, Carbonyl, C₁-C₁₂-Alkylcarboxy, Thiol, C₁-C₁₂-Acyl, gegebenenfalls substituiertes C₁-C₁₂-Aminocarbonyl, C₁-C₁₂-Acyloxycarbonyl oder C₁-C₁₂-Alkylthio.

Gleiches gilt für den Arylteil eines Arylalkyl-Restes.

Beispielsweise steht Aryl besonders bevorzugt für Phenyl, Naphthyl oder Anthracenyl, das gegebenenfalls einfach, zweifach oder dreifach durch Reste substituiert ist, die jeweils unabhängig voneinander ausgewählt sind aus der Gruppe C₁-C₆-Alkyl, C₁-C₆-Halogenalkyl, C₅-C₁₄-Aryl, C₁-C₆-Alkoxy, C₁-C₆-Alkoxycarbonyl, Halogen, Hydroxy, Nitro oder Cyano.

**Alkyl** bzw. **Alkylen** bzw. **Alkoxy** steht im Rahmen der Erfindung sofern nicht gesondert vermerkt bevorzugt jeweils unabhängig für einen gegebenenfalls substituierten geradkettigen, cyclischen, verzweigten oder unverzweigten Alkyl- bzw. Alkylen- bzw. Alkoxy-Rest,. Gleiches gilt für den Alkylenteil eines Arylalkyl-Restes. Als Substituenten für die Alkyl- bzw. Alkylen- bzw. Alkoxyreste kommen beispielweise C₁-C₆-Alkyl, C₁-C₆-Halogenalkyl, C₅-C₁₄-Aryl, C₆-C₁₅-Arylalkyl, C₁-C₆-Alkoxy, C₅-C₁₄-Aryloxy, C₁-C₆-Alkoxycarbonyl, C₁-C₆-Acyloxy, gegebenenfalls substituiertes C₁-C₁₂-Alkylamino oder C₁-C₁₂-Alkoxycarbonylamino, wie z.B. tert-Butoxycarbonylamino (BOC-Amino), Carboxyl, Carbonyl, C₁-C₁₂-Alkylcarboxy, C₁-C₁₂-Acyl, gegebenenfalls substituiertes C₁-C₁₂-Aminocarbonyl, C₁-C₁₂-Acyloxycarbonyl oder C₁-C₁₂-Alkylthio, Halogen, Hydroxy, Nitro oder Cyano in Frage.

Beispielsweise steht Alkyl besonders bevorzugt für gegebenenfalls substituiertes Methyl, Ethyl, n-Propyl, Isopropyl, n-Butyl, tert.-Butyl, n-Pentyl, Cyclohexyl und n-Hexyl, n-Heptyl, n-Octyl, isoOctyl, n-Decyl und n-Dodecyl.

Beispielsweise steht Alkylen bevorzugt für gegebenenfalls substituiertes Methylen, 1,1-Ethylen, 1,2-Ethylen, 1,1-Propylen, 1,2-Propylen, 1,3-Propylen, 1,1-Butylen, 1,2-Butylen, 2,3-Butylen und 1,4-Butylen, 1,5-Pentylen, 1,6-Hexylen, 1,1-Cyclohexylen, 1,4-Cyclohexylen, 1,2-Cyclohexylen und 1,8-Octylen.

Beispielsweise steht Alkoxy bevorzugt für Methoxy, Ethoxy, Isopropoxy, n-Propoxy, n-Butoxy, tert.-Butoxy und Cyclohexyloxy.

Cyclische Alkylreste können sowohl 3 bis 7-gliedrige Homo- als auch Heterocyclen mit ingesamt 3 bis 20 Kohlenstoffatomen, letztere vorzugsweise mit 1, 2 oder 3 Heteroatomen, sein. Homocyclische Alkylreste sind beispielsweise gegebenenfalls substituiertes Cyclopentyl oder Cyclohexyl, Beispiele für heterocyclische Alkylreste sind Dioxolan- oder Phthalimidreste.

**Arylalkyl** steht im Rahmen der Erfindung sofern nicht gesondert vermerkt bevorzugt jeweils unabhängig für einen geradkettigen, cyclischen, verzweigten oder unverzweigten Alkylrest, der einfach oder mehrfach, besonders bevorzugt einfach durch Arylreste gemäß vorstehender Definition substituiert ist. Ein Beispiel für einen Arylalkylrest ist Benzyl.

**Halogenalkyl** bzw. **Halogenalkylen** steht im Rahmen der Erfindung sofern nicht gesondert vermerkt bevorzugt jeweils unabhängig für einen geradkettigen, cyclischen, verzweigten oder unverzweigten Alkylrest, der mit einem, mehreren oder vollständig mit Halogenatomen unabhängig voneinander ausgewählt aus der Gruppe Fluor, Chlor, Brom und Iod substituiert sein kann.

Beispielsweise steht C₁-C₈-Halogenalkyl besonders bevorzugt für Trifluormethyl, Trichlormethyl, 2,2,2-Trifluorethyl, Pentafluorethyl und Nonafluorbutyl.

**Halogen** kann für Fluor, Chlor, Brom oder Iod, bevorzugt für Fluor oder Chlor stehen.

Im Folgenden werden bevorzugte Verbindungen der Formeln (I), (II) und (III) definiert.

In den Formeln (I) und (II) stehen R¹ für Wasserstoff und R² für gegebenenfalls substituiertes C₁-C₂₀-Alkyl, gegebenenfalls substituiertes C₁-C₂₀-Alkenyl, gegebenenfalls substituiertes C₅-C₁₄-Aryl, gegebenenfalls substituiertes C₆-C₁₅-Arylalkyl, gegebenenfalls substituiertes C₁-C₂₀-Halogenalkyl, gegebenenfalls substituiertes C₃-C₂₀-Cycloalkyl oder gegebenenfalls substituiertes C₃-C₂₀-Cycloalkenyl.

Besonders bevorzugt sind dabei Verbindungen der Formel (I) und (II), in denen R¹ für Wasserstoff, steht und R² für gegebenenfalls substituiertes C₅-C₁₄-Aryl, für gegebenenfalls substituiertes C₁-C₂₀-Alkyl oder für gegebenenfalls substituiertes C₁-C₂₀-Alkenyl steht. R¹ steht für Wasserstoff. In bevorzugten Ausführungsformen der Erfindung steht R¹ für Wasserstoff und R² für gegebenenfalls substituiertes C₅-C₁₄-Aryl.

Besonders bevorzugte Substituenten für den Rest R² sind dabei Methyl, Ethyl, Methoxy, Ethoxy, Fluor, Chlor, Trifluormethyl, Nitro, Phenyl, Phenoxy und tert-Butoxycarbonylamino (BOC-Amino). Für den Fall, dass es sich bei den Alkoholen der allgemeinen Formel (II) um Polyalkohole der allgemeinen Formel (II-a) handelt, ist ein weiterer bevorzugter Substituent für den Rest R^{2'} Hydroxy und für den Rest R² Carbonyl.

In den Formeln (IIIa) und (IIIb) steht Träger bevorzugt für einen porösen anorganischen Festkörper. Besonders bevorzugt steht Träger für einen porösen anorganischen Festkörper aus der Gruppe Aktivkohle, Aluminiumoxid, Aluminiumsilicat, Siliciumdioxid, Bariumsulfat, Calciumcarbonat, Cerdioxid, Titandioxid und Zirkon(ium)dioxid. Besonders bevorzugt steht Träger für Aktivkohle, Titandioxid und Zirkon(ium)dioxid.

In der Formel (IIIb) steht ÜM bevorzugt für ein Übergangsmetall und besonders bevorzugt für ein Übergangsmetall aus der Gruppe Cu, Mo, Mn, Fe, Co.

Das erfindungsgemäße Verfahren wird in einer bevorzugten Ausführungsform in Gegenwart von Wasser und/oder einem oder mehreren organischen Lösungsmitteln wie insbesondere tertiären Alkoholen, aprotisch polaren Lösungsmitteln, Ketonen, chlorierten Kohlenwasserstoffen und aromatischen Kohlenwasserstoffen durchgeführt. Unter aprotischen polaren Lösungsmittel sind solche zu verstehen, die bei 25°C eine Dielektrizitätskonstante von 5 oder mehr und einen pKs-Wert bezogen auf eine wässrige Bezugsskala bei 25°C von 20 oder mehr aufweisen. Für das erfindungsgemäße Verfahren besonders bevorzugt sind Wasser, tertiäre Alkohole wie insbesondere *t*-Amylalkohol und *t*-Butylalkohol, sowie chlorierte Kohlenwasserstoffe, wie z.B. 1,2-Dichlorethan, Methylenchlorid, Chloroform, Chlorbenzol und Dichlorbenzole, z.B. 1,2-, 1,3-oder 1,4-Dichlorbenzol. In einer ganz besonders bevorzugten Ausführungsform wird das Verfahren zweiphasig, insbesondere bevorzugt in Gegenwart von Wasser und 1,2-Dichlorethan durchgeführt.

Die Reaktion wird beispielsweise so durchgeführt, dass die Verbindungen der Formel (I) und der Rutheniumkatalysator in einem Lösungsmittel vorgelegt und mit dem Alkali- oder Erdalkalimetallhypochlorit, welches gegebenenfalls in Wasser gelöst ist, versetzt werden und über einen Zeitraum von bis zu 24 Stunden, bevorzugt bis zu 5 Stunden und besonders bevorzugt 1 Stunde gerührt werden. In einer bevorzugten Ausführungsform wird eine Lösung des Oxidationsmittels über einen Zeitraum von 5 Minuten bis 24 Stunden, bevorzugt von 5 Stunden und besonders bevorzugt von 1 Stunde zum Reaktionsgemisch zudosiert. Eine etwaige zusätzliche Nachrührzeit kann beispielsweise bis zu 24 Stunden, bevorzugt bis zu 5 Stunden und besonders bevorzugt bis zu 1 Stunde betragen.

Die Reaktion kann bei Temperaturen von -20°C bis 150°C durchgeführt werden, bevorzugt bei 0 bis 80°C, besonders bevorzugt bei 0°C bis 40°C und ganz besonders bevorzugt bei 15°C bis 30°C.

Der Druck bei der Reaktion ist unkritisch und kann beispielsweise 0,5 bis 100 bar, bevorzugt 0,8 bis 10 bar betragen. Besonders bevorzugt ist Umgebungsdruck.

Als als Oxidationsmittel verwendete Alkali- und Erdalkalimetallhypochlorite werden bevorzugt Natrium- oder Kaliumhypochlorit oder Calcium-hypochlorit-chlorid - letzteres beispielsweise in reiner Form oder in Form von technischem Chlorkalk - eingesetzt. Besonders bevorzugt wird Natriumhypochlorit eingesetzt. Das Oxidationsmittel wird bevorzugt in einer Menge von 0.5 bis 10 Moläquivalenten bezogen auf Verbindungen der Formel (II), besonders bevorzugt von 1 bis 5 Moläquivalenten und ganz besonders bevorzugt von 1 bis 3 Moläquivalenten eingesetzt. Das Oxidationsmittel wird gegebenenfalls vorteilhaft als Lösung und/oder Suspension in einem Lösungsmittel eingesetzt, besonders bevorzugt als Lösung und/oder Suspension in Wasser und gegebenenfalls zusätzlich wenigstens einem der vorangehend aufgeführten organischen Lösungsmittel.

Die Reaktion kann unter pH-neutralen sowie unter sauren oder alkalischen Bedingungen durchgeführt werden, gegebenenfalls kann auch der Zusatz von Säuren oder Basen vorteilhaft sein. Bevorzugt wird die Reaktion unter alkalischen Bedingungen durchgeführt, besonders bevorzugt bei pH-Werten von 10 bis 13, gemessen bei 20°C.

Die Menge der eingesetzten anorganisch geträgerten Rutheniumverbindung, insbesondere solcher der Formel (IIIa) und (IIIb), kann so gewählt werden, dass die Menge an Ruthenium bezogen auf den Alkohol der Formel (II) zwischen 0.0001 und 100 mol%, bevorzugt zwischen 0.01 und 10 mol% und besonders bevorzugt zwischen 0.5 und 1 mol% liegt.

Der anorganisch geträgerte Rutheniumkatalysator kann beispielsweise in einfacher Weise durch Filtration oder Zentrifugation vom Reaktionsgemisch abgetrennt werden. Gegebenenfalls kann er mit Wasser und/oder organischem Lösungsmittel gewaschen werden, bevor er wieder erneut in die Reaktion eingesetzt werden kann.

Es kann vorteilhaft sein der Reaktion geringe Mengen geeigneter Radikalfänger zuzugeben. Solche geeigneten Radikalfänger sind dem Fachmann bekannt; beispielhaft sei Hydrochinon genannt. Bevorzugt ist ein Zusatz von Mengen an Radikalfängern von bis zu einem Äquivalent bezogen auf den Metallgehalt des Katalysators. Durch die Zugabe solcher Radikalfänger kann eine etwaige Überoxidation zur Carbonsäure vermieden werden, jedoch ist eine solche Zugabe nicht zwingend erforderlich.

Auf erfindungsgemäße Weise können Verbindungen der Formel (I) mit sehr hohen Selektivitäten unter milden Bedingungen erhalten werden. Die Aufarbeitung kann in an sich bekannter Weise z.B. durch Extraktion mit einem geeigneten organischen Lösungsmittel und Destillation oder Umkristallisation des Aldehyds erfolgen. Nicht umgesetztes Edukt kann in den Prozess zurückgeführt werden.

Im Rahmen der Erfindung werden heterogene, anorganisch geträgerte Ruthenium-Katalystoren eingesetzt, die gegebenenfalls zusätzlich mit einem oder mehreren Übergangsmetallen dotiert sein können.

Der Rutheniumgehalt kann zwischen 0.01 und 20 Gew.%, bevorzugt zwischen 0.05 und 5 Gew.% liegen. Der Gehalt an gegebenenfalls zusätzlich vorhandenem Übergangsmetall oder den gegebenenfalls zusätzlich vorhandenen Übergangsmetallen kann zwischen 0.01 und 20 Gew.%, bevorzugt zwischen 0.1 und 5 Gew.% liegen. Ruthenium-Gehalt und Übergangsmetall-Gehalt beziehen sich auch die Gesamtmasse des Katalysators. Der Gehalt des jeweiligen Metalls kann beispielsweise mittels ICP-OES (inductively coupled plasma - optical emission spectroscopy) bestimmt werden. Die Bestimmung kann beispielsweise auf einem Optima 3000 XL Gerät der Firma Perkin Elmer und einem EA 1110-Gerät der Firma CE Instruments erfolgen.

In den Katalysatoren, insbesondere der Formeln (IIIa) und (IIIb), kann Ruthenium in den formalen Oxidationsstufen 0 bis +VII vorliegen, bevorzugt sind die Oxidationsstufen 0 und +III und besonders bevorzugt ist die Oxidationsstufe +III. Der Oxidationszustand von Ruthenium kann beispielsweise durch XPS ermittelt werden (Bindungsenergie des [Ru3d_{5/2}-Levels]). Die XPS-Messungen können beispielweise an einem VG ESCALAB 220 i XL mit AlK_{α}-Strahlung (1486.8 eV) durchgeführt werden. Solche Geräte sind dem Fachmann bekannt. Zur Bestimmung der Elektronenbindungsenergie wurde als Bezugspunkt das Cls-Signal bei 284.4 eV gewählt.

In bevorzugten Ausführungsformen des erfindungsgemäßen Verfahrens liegt in den Katalysatoren der Formeln (IIIa) und (IIIb) der Durchmesser der Ruthenium-Partikel, welcher durch Transmissions-Elektronenmikroskopie (TEM) bestimmt werden kann, auf dem Träger bei Werten von 20 nm und kleiner. In besonders bevorzugten Ausführungsformen des erfindungsgemäßen Verfahrens liegt der Durchmesser der Ruthenium-Partikel auf dem Träger bei Werten von 2 nm und kleiner. Die TEM-Messungen können beispielsweise mit einem CM-20 Phillips-Gerät bei 200 kW Beschleunigungsleistung durchgeführt werden.

Hinsichtlich der Eigenschaften, wie z.B. der BET-Oberfläche, der als anorganische Träger verwendeten Materialien liegen keine Einschränkungen vor. Als Titandioxide können beispielsweise P25 (BET-Oberfläche 50 m²/g ± 15 m²/g) der Firma Degussa oder TiO₂ der Firma Kronos (BET-Oberfläche 307 m²/g) eingesetzt werden. Als Zirkondioxid kann beispielsweise Zr(OH)₄ MELCAT XZ0631/01 der Firma MEL Chemicals (BET-Oberfläche 459 m²/g) eingesetzt werden. Die Bestimmung der BET-Oberflächen kann beispielsweise mittels eines ASAP 2000-Mg als Adsorptionssystem bei 77 °C erfolgen. Ein solches Gerät ist dem Fachmann bekannt.

Die Herstellung der anorganisch geträgerten Rutheniumkatalysatoren, die gegebenenfalls mit einem oder mehreren Übergangsmetallen dotiert sein können, mit der allgemeinen Formel (IIIa) oder (IIIb) kann beispielsweise in Anlehnung an Mizuno et al., Angew. Chem. 2002, 114, 4720, nasschemisch durch Imprägnierung in der Weise erfolgen, dass eine bestimmte Menge einer Ruthenium(III)-Vorstufe, wie beispielsweise Ruthenium(III)-chlorid, sowie gegebenenfalls eine bestimmte Menge einer oder mehrerer Übergangsmetallverbindung(en), wie beispielsweise Eisen(II)-sulfat oder Cobalt(II)-chlorid, in einem geeigneten Lösungsmittel, wie beispielsweise Wasser, vorgelegt werden und unter Rühren der Träger zugesetzt wird. Danach trennt man den Feststoff von der flüssigen Phase ab, beispielsweise durch Filtration oder Zentrifugation. Der Feststoff wird gegebenenfalls mit Wasser gewaschen und getrocknet. Danach wird der Feststoff in Wasser suspendiert und die erhaltene Mischung wird, beispielsweise mit verdünnter Natronlauge, alkalisch gestellt. Nach mehrstündigem Rühren trennt man den Feststoff von der flüssigen Phase ab, beispielsweise durch Filtration oder Zentrifugation. Der Feststoff wird gegebenenfalls mit Wasser gewaschen und getrocknet.

Nach einer modifizierten nasschemischen Darstellungsweise können anorganisch geträgerte Rutheniumkatalysatoren der Formel (IIIb), die mit einem oder mehreren Übergangsmetallen dotiert sind, auch so hergestellt werden, dass die Übergangsmetallverbindung(en) in Wasser vorgelegt wird (werden) und unter Rühren ein anorganisch geträgerter Rutheniumkatalysator zugesetzt wird. Die weitere Vorgehensweise mit Feststoffabtrennung, Wäsche, Trocknung, Suspendieren in Wasser, Einstellen eines alkalischen pH-Wertes, Feststoffabtrennung, Wäsche und Trocknung erfolgt wie im vorangehenden Abschnitt beschrieben.

Die Herstellung der anorganisch geträgerten Rutheniumkatalysatoren, die gegebenenfalls mit einem oder mehreren Übergangsmetallen dotiert sein können, mit der allgemeinen Formel (IIIa) oder (IIIb), kann auch durch die MPECVD-Methode (microwave plasma-enhanced chemical vapor deposition) erfolgen, beispielsweise in einem Mikrowellen-Plasma-Gerät Ilmplac 1200. Diese Herstellungsvariante kann beispielsweise so erfolgen, dass eine bestimmte Menge einer Ruthenium(III)-Vorstufe, wie beispielsweise Ruthenium(III)-acetylacetonat, sowie gegebenenfalls eine bestimmte Menge einer oder mehrerer Übergangsmetallverbindung(en), wie beispielsweise CobaIt(II)-acetylacetonat im MPECVD-Gerät zusammen mit einer bestimmten Menge des Trägers vorgelegt werden. Die Abscheidung der Ruthenium-Spezies sowie gegebenenfalls der weiteren Übergangsmetall-Species auf dem Träger kann beispielsweise in einem Niederdruck-Sauerstoffplasma erfolgen, gegebenenfalls kann anschließend mit einem Wasserstoffplasma behandelt werden.

Beispiele für Katalysatoren der Formel (IIIa) und (IIIb) sind die Verbindungen der Formeln (III-1) bis (III-12). In den Abkürzungen für die geträgerten Katalystoren steht W für die nasschemische und P für die plasmachemische Herstellungsmethode, die Zahl nach dem jeweiligen Metall, das auf dem Träger aufgebracht ist, gibt die Beladung dieses Metalls in Gewichtsprozent an, bezogen auf die Gesamtmasse des Katalysators. Die Angabe in Klammern ist die kommerzielle Bezeichnung des Trägermaterials.

| **Nummerierung** | **Katalysator** |
|---|---|
| III-1 | W-Ru 0.83/TiO₂ (Kronos) |
| III-2 | W-Ru 0.50/TiO₂ (P25) |
| III-3 | W-Ru 1.36/TiO₂ (P25) |
| III-4 | W-Ru 1.57/TiO₂ (P25) |
| III-5 | W-Ru 1.9/TiO₂ (P25) |
| III-6 | W-Ru 2.13/ZrO₂ |
| III-7 | W-Ru 1.85/Cu 0.18/TiO₂ (P25) |
| III-8 | W-Ru 1.1/Fe 0.30/TiO₂ (P25) |
| III-9 | W-Ru 0.45/Co 0.36/TiO₂ (P25) |
| III-10 | W-Ru 0.44/Mn 0.35/TiO₂ (P25) |
| III-11 | P-Ru 0.2/TiO₂ (P25) |
| III-12 | P-Ru 0.34/TiO₂ (P25) |
| III-13 | P-Ru 1.57/TiO₂ (P25) |
| III-14 | P-Ru 0.32/Co 0.51/TiO₂ (P25) |

Ferner wurden kommerziell erhältliche geträgerte Ruthenium/Aktivkohle-Katalysatoren eingesetzt:
III-15: H 101 R/W (5 % Ru/C; Degussa)
III-16: Escat 440 (5 % Ru/C; Engelhard)
III-17: K-0402 (5 % Ru/C; Heraeus)

Ferner wurde der kommerzielle erhältliche geträgerte Ruthenium/Al₂O₃-Katalysator eingesetzt:
III-18: K-0453 (5 % Ru/Al₂O₃; Heraeus)

Weiterhin Verwandbar sind anorganisch geträgerte Rutheniumkatalysatoren, bevorzugt der allgemeinen Formel (IIIa) oder (IIIb), worin der Durchmesser der Ruthenium-Partikel auf dem Träger bei Werten von 20 nm und kleiner, bevorzugt 5 nm und kleiner, besonders bevorzugt 2 nm und kleiner liegt. Die Durchmesser der Ruthenium-Partikel auf dem Träger können durch Transmissions-Elektronenmikroskopie (TEM) bestimmt werden. Bevorzugt handelt es sich bei den erfindungsgemäßen anorganisch geträgerten Rutheniumkatalysatoren um solche, bei denen als Träger Titandioxid oder Zirkoniumdioxid verwendet werden.

Für die Katalysatoren gelten des Weiteren die vorangehend aufgeführten Definitionen und Vorzugsbereiche für die im erfindungsgemäß eingesetzten anorganisch geträgerten Rutheniumkatalysatoren, insbesondere der allgemeinen Formel (IIIa) und (IIIb), sowie die Angaben zu deren Herstellung.

Die Katalysatoren eignen sich , für die Anwendung im vorangehend beschriebenen erfindungsgemäßen Verfahren.

Die erfindungsgemäß herstellbaren Verbindungen der Formel (I) eignen sich insbesondere zur Herstellung von pharmazeutischen oder agrochemischen Wirkstoffen, Polymeren, Spezialchemikalien oder Zwischenprodukten davon.

Das erfindungsgemäße Verfahren zeichnet sich dadurch aus, dass die Oxidation von primären Akoholen unter sehr milden Bedingungen bei hoher Chemoselektivität verläuft und die entsprechenden Aldehyde in sehr hohen Selektivitäten liefert. Besonders zu erwähnen sind die sehr geringen benötigten Mengen an anorganisch geträgertem Ruthenium-Katalysator, der in einfacher Weise vom Reaktionsgemisch abgetrennt und erneut für die Reaktion eingesetzt werden kann. Gleichzeitig ist die Möglichkeit der Verwendung der preisgünstigen Alkali- oder Erdalkalimetallhypochlorite, insbesondere Natriumhypochlorit (Chlorbleichlauge), als Oxidationsmittel ein besonderer Vorteil.

Die folgenden Beispiele dienen der beispielhaften Erläuterung der Erfindung und sind nicht als Beschränkung aufzufassen.

### Beispiele

Die Bindungsenergie des [Ru3d_{5/2}-Levels]) wurde mit XPS-Messungen an einem VG ESCALAB 220 i XL mit AlK_{α}-Strahlung (1486.6 eV) bestimmt. Zur Bestimmung wurde als Bezugspunkt das Cls-Signal bei 284.4 eV gewählt.

Der Durchmesser der Ruthenium-Partikel wurde mittels TEM-Messungen mit einem CM-20 Phillips-Gerät bei 200 kW Beschleunigungsleistung bestimmt.

Der Gehalt des jeweiligen Metalls (Metallbeladung) wurde mittels ICP-OES auf einem Optima 3000 XL Gerät der Firma Perkin Elmer bzw. einem EA 1110-Gerät der Firma CE Instruments bestimmt.

Die Bestimmung der BET-Oberflächen erfolgte mittels eines ASAP 2000-Mg als Adsorptionssystem bei 77 °C.

### Beispiel 1: Allgemeine Herstellvorschrift der Katalysatoren 111-1 bis III-8.

RuCl₃ oder RuCl₃xH₂O wurden, gegebenenfalls zusammen mit einer Übergangsmetallverbindung, in einer bestimmten Menge Wasser gelöst und unter Rühren der Träger zugesetzt. Die Suspension wurde 15 min gerührt und dann bei 7000 Upm zentrifugiert. Die überstehende Flüssigkeit wurde abdekantiert, der feste Rückstand mit Wasser gewaschen und erneut zentrifugiert. Diese Waschprozedur wurde noch zweimal wiederholt, so dass man eine farblose Waschlauge erhielt. Der Feststoff wurde bei Raumtemperatur im Vakuum 24 bis 48 h lang getrocknet und dann in einer Reibschale zermahlen. Der Feststoff wurde dann in 90 - 180 ml Wasser suspendiert und das Gemisch mit wässriger Natronlauge (1 molar) auf einen pH-Wert von 13.2 eingestellt. Nach 24-stündigem Rühren wurde die Mischung zentrifugiert und der feste Rückstand dreimal mit Wasser gewaschen. Der Feststoff wurde 24 h lang bei Raumtemperatur und zusätzlich 4 h lang bei 50 °C unter Vakuum getrocknet.
Katalysator III-1: 103 mg RuCl₃; 2 g TiO₂ (Kronos); 60 ml Wasser; Ausbeute 1.87 g.
Katalysator III-2: 152 mg RuCl₃·xH₂O; 10 g TiO₂ (P25); 180 ml Wasser; Ausbeute 8.12 g.
Katalysator III-3: 304 mg RuCl₃·xH₂O; 10 g TiO₂ (P25); 180 ml Wasser; Ausbeute 7.99 g.
Katalysator III-4: 516 mg RuCl₃; 10 g TiO₂ (P25); 180 ml Wasser; Ausbeute 8.20 g.
Katalysator III-5: 516 mg RuCl₃; 10 g TiO₂ (P25); 300 ml Wasser; Ausbeute 7.33 g.
Katalysator III-6: 310 mg RuCl₃; 6 g ZrO₂; 180 ml Wasser; Ausbeute 4.87 g.
Katalysator III-7: 103 mg RuCl₃; 10 mg CuCl; 2 g TiO₂ (P25); 60 ml Wasser; Ausbeute 1.85 g.
Katalysator III-8: 103 mg RuCl₃; 32 mg FeSO₄·x7H₂O; 2 g TiO₂ (P25); 60 ml Wasser; Ausbeute 1.49 g.

**Tab. 1: Charakterisierung der Katalysatoren III-1 bis III-8**

| Nummerierung | Metallbeladung [Gew.%] | Durchmesser der Ru-Partikel [nm] | BET-Oberfläche [m²/g] | Bindungsenergie [eV] |
|---|---|---|---|---|
| III-1 | Ru 0.83 | ≤ 1 | 253.8 | [Ru3d_{5/2}] 282.5 |
| III-2 | Ru 0.50 | ≤ 2 | 55.4 | [Ru3d_{5/2}] 282.1 |
| III-3 | Ru 1.36 | n.b. | 53.6 | n.b. |
| III-4 | Ru 1.57 | 1 - 5 | 57.9 | [Ru3d_{5/2}] 282.1 |
| III-5 | Ru 1.90 | ≤ 1-2 | 62.8 | [Ru3d_{5/2}] 281.5 |
| III-6 | Ru 2.13 | ≤ 2 | 433.5 | [Ru3d_{5/2}] 281.8 |
| III-7 | Ru 1.85; Cu 0.18 | n.b. | 57.2 | [Ru3d_{5/2}] 281.8 [Cu2p_{3/2}] 929.7 |
| III-8 | Ru 1.1; Fe 0.30 | n.b. | 55.0 | n.b. |

| | | | | |
|---|---|---|---|---|
| n.b. = nicht bestimmt | | | | |

Die ermittelten Bindungsenergien liegen im des charakteristischen Bereich für Ruthenium der Oxidationsstufe +III.

### Beispiel 2: Herstellung der Katalysatoren III-9 und III-10.

Eine bestimmte Menge einer Cobaltverbindung bzw. einer Mangan-Verbindung wurde in Wasser gelöst und unter Rühren 4 g des Katalysators III-2 [W-Ru 0.50/TiO₂ (P25)] zugesetzt. Die Suspension wurde 24 h gerührt und dann bei 7000 Upm zentrifugiert. Die überstehende Flüssigkeit wurde abdekantiert, der feste Rückstand mit Wasser gewaschen und erneut zentrifugiert. Diese Waschprozedur wurde noch zweimal wiederholt. Der Feststoff wurde bei Raumtemperatur im Vakuum 25 h lang getrocknet und dann in einer Reibschale zermahlen. Der Feststoff wurde dann in 90 ml Wasser suspendiert und das Gemisch mit wässriger Natronlauge (1M) auf einen pH-Wert von 13.2 eingestellt. Nach 24-stündigem Rühren wurde die Mischung zentrifugiert und der feste Rückstand dreimal mit Wasser gewaschen. Der Feststoff wurde 24 h lang bei Raumtemperatur unter Vakuum getrocknet.
Katalysator III-9: 292 mg Co(OAc)₂ 4H₂O; Ausbeute 3.47 g.
Katalysator III-10: 288 mg Mn(OAc)₂·4H₂O; Ausbeute 3.34 g.

**Tab. 2: Charakterisierung der Katalysatoren III-9 und III-10**

| Nummerierung | Metallbeladung [Gew.%] | Durchmesser der Ru-Partikel [nm] | BET-Oberfläche [m²/g] | Bindungsenergie [eV] |
|---|---|---|---|---|
| III-9 | Ru 0.45; Co 0.36 | ≤ 2 | 52.9 | [Ru3d_{5/2}] 282.1 [Co2p_{3/2}] 780.5 |
| III-10 | Ru 0.44; Mn 0.35 | ≤ 2 | 58.5 | [Ru3d_{5/2}] 282.2 [Mn2p_{3/2}] 640.9 |

| | | | | |
|---|---|---|---|---|
| n.b. = nicht bestimmt | | | | |

Die ermittelten Bindungsenergien liegen im charakteristischen Bereich für Ruthenium der Oxidationsstufe +III.

### Beispiel 3: Plasmachemische Herstellung der Katalysatoren III-11 bis III-14.

Eine bestimmte Menge Ruthenium(III)-acetylacetonat sowie im Falle des Katalysators III-14 zusätzlich eine bestimmte Menge Cobalt(II)-acetylacetonat wurden zusammen mit 3 g Titandioxid (P25) im MPECVD-Gerät (Mikrowellen-Plasma-Gerät Ilmplac 1200) vorgelegt. Anschließend wurde ein Vakuum von 10 Pa eingestellt. Das Plasma wurde bei einem Sauerstofffluss von 300 cm³/min und einer Mikrowellenleistung von 300 W gezündet. Katalysator III-13 wurde zusätzlich mit einem Wasserstoffplasma behandelt.
Katalysator III-11: 84 mg Ru(III)-acetylacetonat
Katalysator III-12: 167 mg Ru(III)-acetylacetonat
Katalysator III-13: 240 mg Ru(III)-acetylacetonat
Katalysator III-14: 120 mg Ru(III)-acetylacetonat; 77 mg Cobalt(II)-acetylacetonat

**Tab. 3: Charakterisierung der Katalysatoren III-11 bis III-14**

| Nummerierung | Metallbeladung [Gew.%] | Durchmesser der Ru-Partikel [nm] | BET-Oberfläche [m²/g] | Bindungsenergie [eV] |
|---|---|---|---|---|
| III-11 | Ru 0.2 | ≤ 5 | 60.2 | [Ru3d_{5/2}] 280.6 |
| III-12 | Ru 0.34 | 1-10 | 59.4 | [Ru3d_{5/2}] 280.3 |
| III-13 | Ru 1.57 | 1.5-10 | 58.7 | (Ru3d_{5/2}] 280.7 |
| III-14 | Ru 0.32; Co 0.51 | 1-10 | 59.6 | [Ru3d_{5/2}] 282.3 [Co2p_{3/2}] 780.5 |

| | | | | |
|---|---|---|---|---|
| n.b. = nicht bestimmt | | | | |

Die ermittelten Bindungsenergien der Katalysatoren III-11, III-12 und III-13 liegen geringfügig oberhalb des charakteristischen Bereichs für Ruthenium der Oxidationsstufe 0, woraus hervorgeht, dass die Katalysatoren III-11, III-12 und III-13 Ruthenium als Hauptanteil in Form metallischen Rutheniums enthalten. Die ermittelte Bindungsenergie des Katalysators III-14 wird durch die Gegenwart des Promotormetalls Co erhöht, so dass der Wert unter Berücksichtigung dieser Erhöhung ebenfalls für Ruthenium der Oxidationsstufe 0, d.h. für einen Hauptanteil metallischen Rutheniums, spricht.

### Beispiel 4: Allgemeine Versuchsbeschreibung zur Oxidation von Alkoholen mit Natriumhypochlorit.

In einem Reaktionskolben wurden 1 mmol des jeweiligen Alkohols in 7 ml des jeweiligen Lösungsmittels vorgelegt und unter Rühren der Katalysator zugesetzt. Als Oxidationsmittel wurde wässrige Natriumhypochlorit-Lösung (Fa. Sigma-Aldrich, 10 - 13 % aktives Chlor) verwendet, welche innerhalb von 60 min mit Hilfe einer Spritzenpumpe zur Reaktionsmischung dosiert wurde. Es wurde eine bestimmte Zeit nachgerührt. Anschließend wurden die organischen Komponenten durch dreimalige Extraktion mit 1,2-Dichlorethan abgetrennt. Die vereinigten organischen Phasen wurden gaschromatographisch analysiert. Die Gaschromatographie erfolgte auf einem Hewlett Packard HP 5890-Gerät, das mit einer HP5-Säule und einem massenselektiven Detektor (HP 5971 A) ausgestattet war. Als interner Standard wurde Diethylenglycol-di-n-butylether verwendet. Um etwaig gebildete Carbonsäuren nachzuweisen, wurde Trimethylsulfoniumhydroxid vor der Messung zu der Analysenprobe gegeben.

Alle in den folgenden Tabellen aufgeführten Experimente wurden gemäß der allgemeinen Versuchsbeschreibung (Beispiel 4) durchgeführt.

**Tab. 4: Oxidation von Benzylalkohol mit verschiedenen Katalysatoren, Lösungsmitteln und NaOCl-Mengen.**

| Katalysator | Ru [mmol] | Solvens | NaOCl [mmol] | Umsatz [%] | Selektivität Aldehyd [%] | Selektivität Benzoesäure [%] |
|---|---|---|---|---|---|---|
| **III-5** | 0.01 | 3.5ml H₂O/ 3.5 ml *t*-BuOH | 1.1 | 44 | >99 | 0 |
| **III-5** | 0.01 | 3.5 ml H₂O/ 3.5 ml *t*-BuOH | 1.1 | 66 | >99 | 0 |
| **III-5** | 0.01 | 7 ml *t*-BuOH | 1.1 | 42 | 90 | 9 |
| **III-5** | 0.01 | 3.5 ml H₂O/ 3.5 ml DCE | 1.1 | 83 | >99 | 0 |
| **III-5** | 0.01 | 3.5 ml H₂O/ 3.5 ml DCE | 1.1^{a} | 76 | 96 | 3 |
| **III-5** | 0.005 | 3.5 ml H₂O/ 3.5 ml DCE | 1.1^{a} | 77 | >99 | 0 |
| **III-1** | 0.01 | 3.5 ml H₂O/ 3.5 ml DCE | 1.1 | 74 | 98 | 1 |
| **III-6** | 0.01 | 3.5 ml H₂O/ 3.5 ml DCE | 1.1 | 77 | 98 | 1 |
| **III-12** | 0.005 | 3.5 ml H₂O/ 3.5 ml DCE | 1.1 | 47 | 50 | 49 |
| **III-7** | 0.01 | 3.5 ml H₂O/ 3.5 ml DCE | 1.1 | 75 | 97 | 3 |
| **III-8** | 0.01 | 3.5 ml H₂O/ 3.5 ml DCE | 1.1 | 72 | 97 | 3 |
| **III-9** | 0.005 | 3.5 ml H₂O/ 3.5 ml DCE | 1.1 | 38 | 50 | 50 |
| **III-10** | 0.005 | 3.5 ml H₂O/ 3.5 ml DCE | 1.1 | 85 | 87 | 13 |
| **III-5** | 0.01 | 3.5 ml H₂O/ 3.5 ml DCE | 1.3 | 85 | 95 | 5 |
| **III-5** | 0.01 | 3.5 ml H₂O/ 3.5 ml DCE | 1.5 | 100 | 86 | 14 |
| **III-5** | 0.01 | 3.5 ml H₂O/ 3.5 ml DCE | 2 | 100 | 74 | 26 |
| **III-1** | 0.01 | 3.5 ml H₂O/ 3.5 ml DCE | 1.5 | 80 | 97 | 2 |
| **III-6** | 0.01 | 3.5 ml H₂O/ 3.5 ml DCE | 1.5 | 90 | 98 | 2 |
| **III-7** | 0.01 | 3.5 ml H₂O/ 3.5 ml DCE | 1.5 | 97 | 94 | 6 |
| **111-15** | 0.01 | 3.5 ml H₂O/ 3.5 ml DCE | 1.1 | 55 | 99 | 1 |
| **III-16** | 0.01 | 3.5 ml H₂O/ 3.5 ml DCE | 1.1 | 57 | 96 | 3 |
| **III-17** | 0.01 | 3.5 ml H₂O/ 3.5 ml DCE | 1.1 | 66 | 99 | 1 |
| **III-18** | 0.005 | 7 ml *t*-BuOH | 1.1 | 19 | 99 | 0 |

| | | | | | | |
|---|---|---|---|---|---|---|
| Reaktionsbedingungen: 1 mmol Benzylalkohol, Raumtemperatur, Nachrührzeit 10 min; ^{a} Nachrührzeit 130 min, DCE = 1,2-Dichlorethan. | | | | | | |

**Tab. 5: Oxidation von Benzylalkohol bei verschiedenen pH-Werten.**

| Katalysator | Ru [mmol] | Solvens | pH-Wert | Umsatz [%] | Selektivität Aldehyd [%] | Selektivität Benzoesäure [%] |
|---|---|---|---|---|---|---|
| **III-5** | 0.01 | 3.5 ml Puffer/ 3.5 ml DCE | 9 | 65 | 95 | 6 |
| **III-5** | 0.01 | 3.5 ml Puffer/ 3.5 ml DCE | 10 | 65 | 96 | 5 |
| **III-5** | 0.01 | 3.5 ml Puffer/ 3.5 ml DCE | 11 | 73 | 95 | 5 |
| **III-5** | 0.01 | 3.5 ml Puffer/ 3.5 ml DCE | 12 | 67 | 96 | 4 |
| **III-5** | 0.01 | 3.5 ml Puffer/ 3.5 ml DCE | 13 | 64 | 95 | 6 |

| | | | | | | |
|---|---|---|---|---|---|---|
| Reaktionsbedingungen: 0.005 mmol Ru, 1 mmol Benzylalkohol, Raumtemperatur, Nachrührzeit 10 min; DCE = 1,2-Dichlorethan. | | | | | | |

**Tab. 6: Oxidation verschiedener Alkohole mit Katalysator III-5 [W-Ru 1.9/TiO₂ (P25)].**

| Alkohol | Umsatz [%] | Selektivität Aldehyd bzw. Keton [%] | Selektivität Carbonsäure [%] |
|---|---|---|---|
| 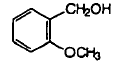 | 62 | 98 | 2 |
| 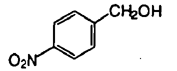 | 100 | 99 | 1 |
| 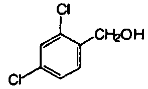 | 62 | 80 | 20 |
| 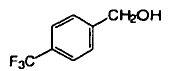 | 55 | 91 | 9 |
| 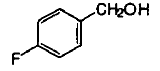 | 67 | 99 | 1 |
| 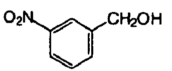 | 35 | 96 | 3 |
| 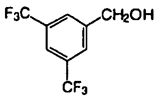 | 58 | >99 | 0 |
| 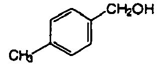 | 73 | 98 | 1 |
| 1-octanol | 36 | 77 | 22 |
| (*E*)-Zimtalkohol | 20 | 37 | 0 |
| *N*-Boc-*D*-Leucinol | 2 | >99 | 0 |
| Cyclohexanol | 33 | 87 | 0 |

| | | | |
|---|---|---|---|
| Reaktionsbedingungen: 0.005 mmol Katalysator III-5; 1 mmol Alkohol, 3.5 ml H₂O/ 3.5 ml 1,2-Dichlorethan, Raumtemperatur, Nachrührzeit 10 min. | | | |

## Patentansprüche

1. Verfahren zur Herstellung von Aldehyden der allgemeinen Formel (I), 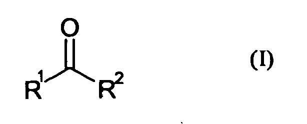 worin
R¹ für ein Wasserstoffatom steht und R²
für einen gegebenenfalls substituierten Kohlenwasserstoffrest, der eine Alkylgruppe, Cycloalkylgruppe, Alkenylgruppe, Arylgruppe, Arylalkylgruppe oder Arylalkenylgruppe mit jeweils 1 bis 20 Kohlenstoffatomen darstellen kann steht oder
für eine gegebenenfalls substituierte heterocyclische Gruppe, die bevorzugt mindestens ein Heteroatom aus der Gruppe Sauerstoff, Stickstoff und Schwefel besitzt,
**dadurch gekennzeichnet, dass** eine oder mehrere Verbindung(en) der allgemeinen Formel (II), 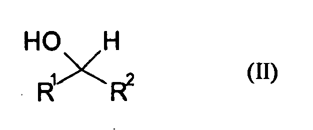 in der R¹ und R² die vorangehend genannten Bedeutungen besitzen,
mit einem Alkali- oder Erdalkalimetallhypochlorit in Gegenwart eines heterogenen, anorganisch geträgerten gegebenenfalls zusätzlich mit einem anderen Übergangsmetall dotierten Ruthenium-Katalysators umgesetzt werden.

2. Verfahren gemäß Anspruch 1, **dadurch gekennzeichnet, dass** als heterogener, anorganisch geträgerter Ruthenium-Katalysator ein solcher der allgemeinen Formel (IIIa) oder (IIIb) verwendet wird
Ru/Träger (IIIa)
worin
Ru/ÜM/Träger (IIIb),
ÜM für ein Übergangsmetall und
Träger für ein anorganisches Trägermaterial stehen.

3. Verfahren gemäß Anspruch 1 oder 2, **dadurch gekennzeichnet, dass** als Träger ein poröser anorganischer Festkörper ausgewählt aus der Gruppe Aktivkohle, Aluminiumoxid, Aluminiumsilicat, Siliciumdioxid, Bariumsulfat, Calciumcarbonat, Cerdioxid, Titandioxid und Zirkoniumdioxid verwendet wird.

4. Verfahren gemäß wenigstens einem der Ansprüche 1 bis 3, **dadurch gekennzeichnet, dass** R¹ für Wasserstoff steht und R² für, gegebenenfalls substituiertes C₁-C₂₀-Alkyl, gegebenenfalls substituiertes C₁-C₂₀-Alkenyl, gegebenenfalls substituiertes C₅-C₁₄-Aryl, gegebenenfalls substituiertes C₆-C₁₅-Arylalkyl, gegebenenfalls substituiertes C₁-C₂₀-Halogenalkyl, gegebenenfalls substituiertes C₃-C₂₀-Cycloalkyl oder gegebenenfalls substituiertes C₃-C₂₀-Cycloalkenyl.

5. Verfahren gemäß wenigstens einem der Ansprüche 1 bis 4, **dadurch gekennzeichnet, dass** R¹ für Wasserstoff steht und R² für substituiertes C₅-C₁₄-Aryl.

6. Verfahren gemäß wenigstens einem der Ansprüche 1 bis 5, **dadurch gekennzeichnet, dass** die Umsetzung in Gegenwart von Wasser und/oder einem oder mehreren organischen Lösungsmitteln wie insbesondere tertiären Alkoholen, aprotisch polaren Lösungsmitteln, Ketonen, chlorierten Kohlenwasserstoffen und aromatischen Kohlenwasserstoffen durchgeführt wird.

7. Verfahren gemäß wenigstens einem der Ansprüche 1 bis 6, **dadurch gekennzeichnet, dass** als Alkali- und Erdalkalimetallhypochlorit Natrium- oder Kaliumhypochlorit oder Calcium-hypochlorit-chlorid verwendet wird.

8. Verfahren gemäß wenigstens einem der Ansprüche 1 bis 7, **dadurch gekennzeichnet, dass** das Alkali- und Erdalkalimetallhypochlorit in einer Menge von 0.5 bis 10 Moläquivalenten bezogen auf die Stoffmenge der Verbindungen der Formel (II) eingesetzt wird.

## Claims

1. Process for preparing aldehydes of the general formula (I), 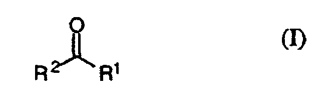 where
R¹ is a hydrogen atom and R² is
a substituted or unsubstituted hydrocarbon radical which may be an alkyl group, cycloalkyl group, alkenyl group, aryl group, arylalkyl group or arylalkenyl group having, in each case, from 1 to 20 carbon atoms or
a substituted or unsubstituted heterocyclic group which preferably has at least one heteroatom from the group consisting of oxygen, nitrogen and sulphur, **characterized in that** one or more compound(s) of the general formula (II), 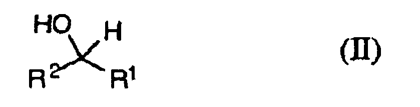 where R¹ and R² are as defined above,
is/are reacted with an alkali metal hypochlorite or alkaline earth metal hypochlorite in the presence of a heterogeneous, inorganically supported ruthenium catalyst which may additionally be doped with another transition metal.

2. Process according to Claim 1, **characterized in that** the heterogeneous, inorganically supported ruthenium catalyst used is a catalyst of the general formula (IIIa) or (IIIb)
Ru/support (IIIa)
Ru/TM/support (IIIb),
where
TM is a transition metal and
support is an inorganic support material.

3. Process according to Claim 1 or 2, **characterized in that** a porous inorganic solid selected from the group consisting of activated carbon, aluminium oxide, aluminium silicate, silicon dioxide, barium sulphate, calcium carbonate, cerium dioxide, titanium dioxide and zirconium dioxide is used as support.

4. Process according to at least one of Claims 1 to 3, **characterized in that** R¹ is hydrogen and R² is substituted or unsubstituted C₁-C₂₀-alkyl, substituted or unsubstituted C₁-C₂₀-alkenyl, substituted or unsubstituted C₅-C₁₄-aryl, substituted or unsubstituted C₆-C₁₅-arylalkyl, substituted or unsubstituted C₁-C₂₀-haloalkyl, substituted or unsubstituted C₃-C₂₀-cycloalkyl or substituted or unsubstituted C₃-C₂₀-cycloalkenyl.

5. Process according to at least one of Claims 1 to 4, **characterized in that** R¹ is hydrogen and R² is substituted C₅-C₁₄-aryl.

6. Process according to at least one of Claims 1 to 5, **characterized in that** the reaction is carried out in the presence of water and/or one or more organic solvents such as, in particular, tertiary alcohols, aprotic polar solvents, ketones, chlorinated hydrocarbons and aromatic hydrocarbons.

7. Process according to at least one of Claims 1 to 6, **characterized in that** sodium hypochlorite or potassium hypochlorite or calcium hypochlorite chloride is used as alkali metal hypochlorite or alkaline earth metal hypochlorite.

8. Process according to at least one of Claims 1 to 7, **characterized in that** the alkali metal hypochlorite or alkaline earth metal hypochlorite is used in an amount of from 0.5 to 10 molar equivalents based on the molar amount of the compounds of the formula (II).

## Revendications

1. Procédé pour la préparation d'aldéhydes de formule générale (I), 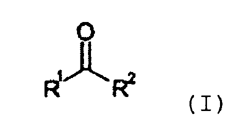 dans laquelle
R¹ représente un atome d'hydrogène et R² représente un radical hydrocarboné éventuellement substitué, qui peut représenter un groupe alkyle, groupe cycloalkyle, groupe alcényle, groupe aryle, groupe arylalkyle ou groupe arylalcényle, ayant chacun de 1 à 20 atomes de carbone ou
représente un groupe hétérocyclique éventuellement substitué, qui comporte de préférence au moins un hétéroatome choisi dans l'ensemble constitué par l'oxygène, l'azote et le soufre,
**caractérisé en ce qu'**on fait réagir un ou plusieurs composé(s) de formule générale (II), 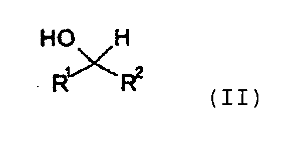 dans laquelle R¹ et R² ont les significations données précédemment,
avec un hypochlorite de métal alcalin ou alcalino-terreux en présence d'un catalyseur hétérogène au ruthénium, fixé sur support inorganique, éventuellement en outre dopé avec un autre métal de transition.

2. Procédé selon la revendication 1, **caractérisé en ce qu'**on utilise comme catalyseur hétérogène au ruthénium, fixé sur support inorganique un tel catalyseur de formule générale (IIIa) ou (IIIb)
Ru/support (IIIa)
où
Ru/MT/support (IIIb)
MT représente un métal de transition et
support représente une matière inorganique de support.

3. Procédé selon la revendication 1 ou 2, **caractérisé en ce qu'**on utilise comme support un corps solide inorganique poreux, choisi dans le groupe constitué par le charbon actif, l'oxyde d'aluminium, le silicate d'aluminium, le dioxyde de silicium, le sulfate de baruym, le carbonate de calcium, le dioxyde de cérium, le dioxyde de titane et le dioxyde de zirconium.

4. Procédé selon au moins l'une des revendications 1 à 3, **caractérisé en ce que** R¹ représente un atome d'hydrogène et R² représente un groupe alkyle en C₁-C₂₀ éventuellement substitué, alcényle en C₁-C₂₀ éventuellement substitué, aryle en C₅-C₁₄ éventuellement substitué, arylalkyle en C₆-C₁₅ éventuellement substitué, halogénoalkyle en C₁-C₂₀ éventuellement substitué, cycloalkyle en C₃-C₂₀ éventuellement substitué ou cycloalcényle en C₃-C₂₀ éventuellement substitué.

5. Procédé selon au moins l'une des revendications 1 à 4, **caractérisé en ce que** R¹ représente un atome d'hydrogène et R² représente un groupe aryle en C₅-C₁₄ substitué.

6. Procédé selon au moins l'une des revendications 1 à 5, **caractérisé en ce que** la réaction est effectuée en présence d'eau et/ou d'un ou plusieurs solvants organiques tels qu'en particulier des alcools, des solvants polaires aprotiques, des cétones, des hydrocarbures chlorés et des hydrocarbures aromatiques.

7. Procédé selon au moins l'une des revendications 1 à 6, **caractérisé en ce qu'**on utilise comme hypochlorite de métal alcalin ou alcalino-terreux l'hypochlorite de sodium ou de potassium ou le chlorure-hypochlorite de calcium.

8. Procédé selon au moins l'une des revendications 1 à 7, **caractérisé en ce qu'**on utilise l'hypochlorite de métal alcalin ou alcalino-terreux en une quantité de 0,5 à 10 équivalents molaires, par rapport à la quantité de substance des composés de formule (II).
